# EUROPEAN PATENT SPECIFICATION

(11) **EP 0 957 868 B1**
(45) Date of publication and mention of the grant of the patent: **05.02.2003**
(21) Application number: 97942610.3
(22) Date of filing: 22.09.1997
(51) Int. Cl.: A61F 13/15

(54) **DISPOSABLE ABSORBENT GARMENT AND METHOD OF CONSTRUCTING THE SAME**
ABSORBIERENDER WEGWERFARTIKEL UND VERFAHREN ZUR HERSTELLUNG DERSELBEN
SOUS-VETEMENT ABSORBANT JETABLE ET PROCEDE DE FABRICATION ASSOCIE

(30) Priority: 15.10.1996 US 730752
(43) Date of publication of application: 24.11.1999
(73) Proprietor: THE PROCTER & GAMBLE COMPANY, Cincinnati, Ohio 45202 (US)
(72) Inventor: JOHNSON, Larry, Kenneth, Cincinnati, OH 45244 (US); LANGE, Stephen, Joseph, Cincinnati, OH 45224-1227 (US)
(74) Representative: Mather, Peter Geoffrey
(86) International application number: US9716707
(87) International publication number: WO98016179

(56) References cited:
- EP-A- 0 508 477
- GB-A- 2 003 390
- US-A- 2 294 898
- US-A- 4 578 066
- US-A- 5 207 664

## Description

### TECHNICAL FIELD

The present invention relates to disposable, absorbent garments and, more particularly, to a disposable garment in which a single, continuous web is utilized as both a top and bottom sheet to increase the strength and leak resistance of the garment, and in which the edges of the folded-over top sheet are raised to provide improved containment of solid waste material.

### BACKGROUND OF THE INVENTION

Today, disposable, absorbent garments are widely used in infant and toddler care, and in the care of incontinent adults, as a means of trapping and disposing of bodily waste. These garments have generally replaced reusable, washable cloth garments as the preferred means for isolating and disposing of waste. The typical disposable garment is a composite structure containing a number of layers of material. Included in these layers of material are a liquid impermeable outer layer or backsheet, one or more layers of woven or non-woven material forming an absorbent core, and a liquid permeable inner layer or top sheet. The layers comprising the garment are generally secured together by lines of adhesive, with the back and top sheets usually directly adhesively interconnected around the periphery of the garment. Elastic bands are provided along the longitudinal sides of the garment to constrict the top and bottom sheets and produce leg cuffs which fit snugly about the wearer's legs. In addition, pairs of closure devices, such as adhesive tabs, may be provided for removably holding the sides of the garment together about the waist of the wearer. Alternatively, the garment may be folded and sealed along opposing side edges to form a pant or brief.

While many advancements have been made in the field of disposable garments for both adults and infants, which have enabled them to be widely preferred over conventional cloth garments, a number of problems still exist. Chief among the problems experienced with today's disposable garments is leakage of liquid waste, particularly along the edges and leg openings in the garment. This leakage soils the wearer's clothing and bedding, leading to additional, cumbersome clothing and bedding changes. The leakage of liquid waste from the garment is due in part to the permeability of the top sheet. Traditionally, the top sheet has extended across the entire top surface of the garment and has typically been joined to the bottom sheet by seams along the edges of the garment. In addition, the top sheet has been made from a liquid permeable or hydrophilic material to allow liquids to pass easily through the sheet to the absorbent core below. The permeable nature of the top sheet has increased the transfer of liquid to the garment core, thus improving the garment's ability to contain liquids. Unfortunately, however, the permeability of the top sheet can also allow liquid to wick throughout the entire top sheet such that it leaks from the edges of the garment.

In addition to the permeable nature of the top sheet, leakage from disposable garments is also caused in part by the poor fit of the garment to the wearer. Many disposable garments are composed of materials with little or no elasticity or stretch across the width of the diaper. This inability to stretch reduces the garment's conformity to the wearer, and leads to gaps between the garment and the wearer. These gaps allow liquid and solid waste to escape the garment and soil the wearer and/or adjacent environment.

In addition to leakage problems, many of the disposable garments available to date lack an effective means within the garment for containing solid waste. Disposable garments have traditionally included a topsheet which is disposed against the wearer's skin along substantially the entire extent of the garment. Because the topsheet is in close contact with the wearer, solid waste that is exuded into the garment remains in contact with the wearer's skin, creating undesirable effects such as skin rashes, discomfort and messy cleanups.

Accordingly, to overcome the above and other problems, it is desirable to have a disposable, absorbent garment which provides superior leak resistance while also improving the strength and fit of the garment. In addition, it is desirable to have such a garment which also provides improved containment of solid waste, in order to increase the wearer's comfort and reduce the clean up time required as part of a garment change.

### SUMMARY OF THE INVENTION

Accordingly, it is a principal object of the present invention to provide a disposable garment for infants and adults which exhibits improved strength and leak resistance.

In particular, it is a principal object of the present invention to provide a disposable, absorbent garment in which a single continuous web is folded-over to form both a top and bottom sheet, to eliminate seams between the sheets.

Another object of the present invention is to provide a disposable garment having a carrier web which is notched prior to being folded-over as the top sheet, in order to form a port in the topsheet for the acquisition and containment of bodily waste.

Yet another object of the present invention is to provide a disposable garment which requires less material to construct than traditional disposable garments, yet provides increased leak protection and containment of waste material.

A further object of the present invention is to provide a disposable garment which has increased stretch across the width of the garment to improve the fit of the garment to the wearer.

Still another object of the present invention is to provide a disposable garment which eliminates leakage along the peripheral edges of the garment.

Yet another object of the present invention is to provide a disposable garment which has superior solid waste containment in order to reduce the time required to clean the wearer during a garment change.

A still further object of the present invention is to provide a garment having the above features which can be worn as either a diaper, a child training pant or an adult incontinence brief.

Additional objects, advantages and other novel features of the invention will be set forth in part in the description that follows and, in part, will become apparent to those skilled in the art upon examination of the invention. The objects and advantages of the invention may be realized and obtained by means of the instrumentalities and combinations particularly pointed out in the appended claims.

To achieve the foregoing and other objects, and in accordance with the purposes of the present invention as described above, a disposable garment is provided comprising an absorbent core and a stretchable, substantially liquid impermeable web. The core is attached to the web by a strip of adhesive extending in a longitudinal direction along the bottom surface of the core. The web is folded around the exterior of the core and includes notches formed in opposing sides of the crotch portion of the web. When folded about the core, the notched areas form a longitudinally extending opening which provides access to the core. A plurality of elastic bands are affixed along the crotch portion of the web, so as to extend substantially parallel to, but inwardly of, the notched areas. The elastic bands constrict the web adjacent the notches to form cuffs which fit snugly about the wearer's legs. Adhesive tabs or another similar closure mechanism are provided on opposite sides of the web to hold the garment together about the wearer's torso. Alternatively, opposing sides of the garment may be sealed together to form a pant. Elastomeric material is affixed at strategic positions on the web to increase the elastic force of the web when stretched, to enable the garment to better conform to the wearer's body. In addition, a lifting arrangement such as heat shrinkable film may be disposed about the web opening formed by the notches, in order to raise the edges of the opening and form a pocket for containing solid waste materials.

Still other objects of the present invention will become apparent to those skilled in this art from the following description wherein there is shown and described a preferred embodiment of this invention, simply by way of illustration, of one of the best modes contemplated for carrying out the invention. As will be realized, the invention is capable of other different, obvious aspects all without departing from the invention.

### BRIEF DESCRIPTION OF THE DRAWINGS

While the specification concludes with claims particularly pointing out and distinctly claiming the present invention, it is believed the same will be better understood from the following description taken in conjunction with the accompanying drawings in which:
Fig. 1 is a perspective view of a first embodiment of the present invention wherein the garment is a diaper;
Fig. 2 is a perspective view of the diaper of Fig. 1, in which a portion of the web is folded back to show the inner structure of the diaper;
Fig. 3 is a top view of the carrier web and core of the diaper in a first, open position;
Fig. 4 is a top view of the carrier web and core of Fig. 3 in a second, folded position;
Fig. 5 is a top plan view similar to Fig. 3, depicting the elastic bands and fold lines for the diaper;
Fig. 6 is a cross-sectional view taken along lines 6-6 of Fig. 1, depicting the carrier web folded about the core;
Fig. 7 is a top plan view of the diaper of Fig. 1, depicting a first embodiment for forming a waste pocket;
Fig. 8 is a top plan view of the diaper of Fig. 1, depicting a second embodiment for forming a waste pocket;
Fig. 9 is a perspective view of an alternative embodiment of the present invention, wherein the garment is a pant or brief; and
Fig. 10 is a perspective view similar to Fig. 1, depicting an alternative embodiment for the garment leg cuffs.

### DETAILED DESCRIPTION OF THE PREFERRED EMBODIMENTS

The disposable, absorbent garment of the present invention may be utilized, with only minor variations, as either a diaper, a child's training pant or an adult incontinence brief. For purposes of the present discussion, the garment will be described in terms of a diaper embodiment. However, it is to be understood that the described features are present in the other embodiments, and, thus, that the invention can be embodied in these other formats without departing from the scope of the invention.

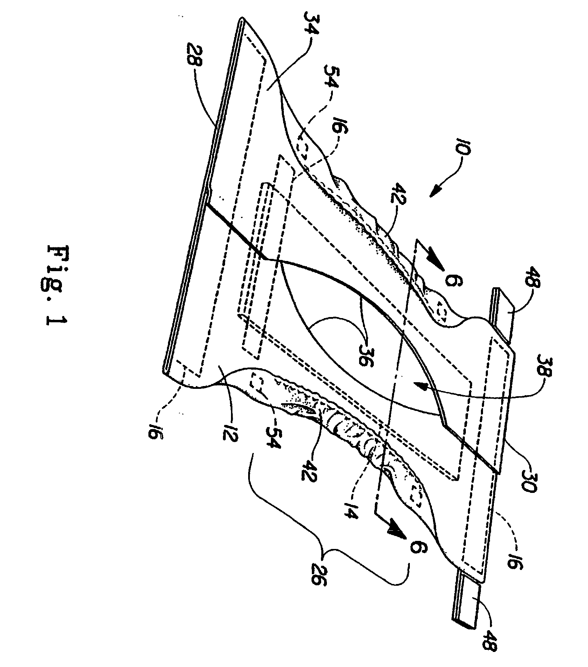

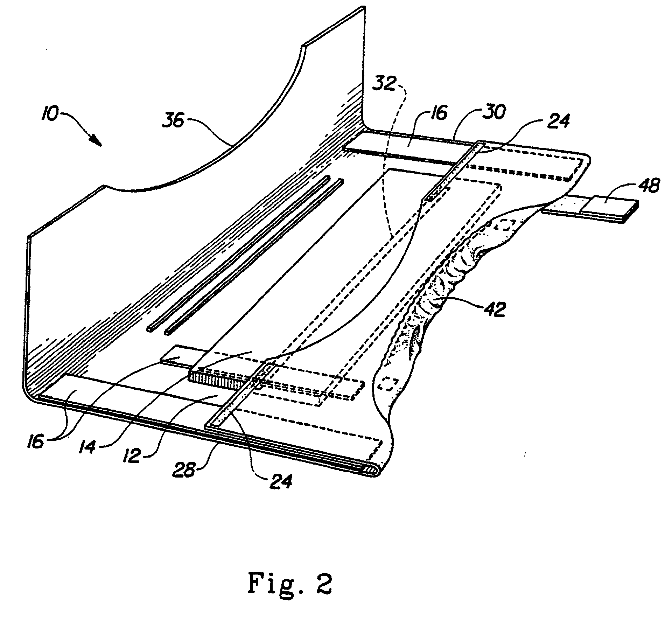

Referring now to the drawings, Figs. 1 and 2 depict the garment of the present invention in a representative diaper embodiment. As shown in these figures, the diaper, designated generally as 10, includes an outer carrier web 12 and an inner absorbent core 14. The carrier web 12 extends along the bottom surface of the diaper 10, and is preferably comprised of a non-woven, liquid impermeable material in order to prevent body fluids, such as urine, from leaking through the bottom surface of the diaper. While the invention has been described with respect to a non-woven web, it is to be understood that a woven web could also be used without departing from the scope of the invention.

In addition, the carrier web 12 is preferably uniformly stretchable in a cross-machine direction across the entire width of the material. In a preferred embodiment, a post-process treatment is performed on the carrier web in order to impart stretchability to the web. While there are several well-known methods that can impart sufficient stretchability into the carrier web, one example, though generally capable of imparting stretchability that could be excessive, would be such as that disclosed in U.S. Patent No. 5,244,482, which is assigned to the University of Tennessee. While the aforementioned patented process uses thermal energy to post treat the carrier web, mechanical energy or chemical treatment methods, may also be used to impart the desired stretch characteristics without departing from the scope of the invention. The use of a uniformly stretchable material for the carrier web 12 enables the web to conform its shape to that of the wearer's body. This enables the diaper 10 to change shape as the wearer moves, reducing gaps and resulting leaks, as well as improving the wearer's comfort.

In order to increase the return force of the web 12 when it is stretched in the cross-machine direction, resilient structure, such as a multi-directional elastomeric material, is associated with the web. In the representative embodiment shown in the figures, the elastomeric material is in the form of strips or patches 16 which are affixed to the inner surface of the web 12. The strips 16 are preferably affixed to the web 12 when neither the strips nor the web are in a strained condition. The locations at which the elastomeric strips 16 are attached to the web 12 are preferably selected so as to increase the elastic return force of the web when stretched. Typically, the elastomeric strips 16 will be applied to the waist and crotch portions of the diaper, as it is usually desirable to have increased elastic force in these areas to assure that the diaper stays in the proper position on the wearer's torso, and that gaps do not form in the crotch area of the diaper. However, the particular locations at which the elastomeric material is applied, and the particular sizes and shapes of the material, are dependent upon the type and use of the garment, as well as the desired degree of fit and conformance of the garment to the wearer's body. Accordingly, elastomeric material may be applied in other forms and at other, different areas of the garment without departing from the scope of the invention.

In addition to the carrier web 12, the garment of the present invention includes an absorbent core 14. The absorbent core 14 is preferably comprised of a number of layers of material, which may have differing characteristics, for collecting and retaining body fluids. The core layers may include a woven or non-woven tissue sheet, a poly sheet, a layer of absorbent material, and an acquisition layer. An example of a core suitable for the present invention is described in U.S. Patent No. 5,246,433, which is assigned to the Assignee of the present invention. The layers described above, as well as those set forth in U.S. Patent No. 5,246,433, are only representative of the types and arrangement of layers which may be utilized in the diaper core. Additional core components can be added, or layers can be deleted or substituted, or the orientation of the layers can be changed, without departing from the scope of the invention, provided an acquisition or other absorbent layer is positioned adjacent the opening in the carrier web as will be described below. An acquisition or other absorbent layer is preferably the topmost layer in the core 14, to assure the highest degree of fluid transfer from the wearer to the other absorbent layers of the core.

As shown in Figs. 1 and 2, in the present invention the carrier web 12 is used as both a top and bottom sheet for the diaper 10. The layers making up the absorbent portion of the diaper are assembled into the core 14, which is attached adjacent the center of the web 12 along the crotch portion 26. As shown in Fig. 1, the core 14 preferably extends longitudinally and substantially front to back through the diaper 10, from just below the front and back waist portions 28, 30, through the crotch area 26, with the length of the core being substantially longer than its width. The core 14 is attached to the web 12 by an attachment device, such as adhesive material 32 depicted in the drawings, to retain the core in proper position on the web. Preferably, the adhesive material is applied in a thin, longitudinally extending band that is generally centered across the width of the core. Utilizing a narrow, centered band of adhesive to attach the core to the web enables the garment to maintain maximum flexibility and stretch, and also enables the core to stretch along with the web. While the band of adhesive used may be as wide as desired, each additional measure of adhesive will restrict the stretch of the garment by up to three times the width of the adhesive band minus the flexibility of the adhesive. Thus, it is preferable to limit the width of the adhesive band to the minimum required to securely attach the core to the web.

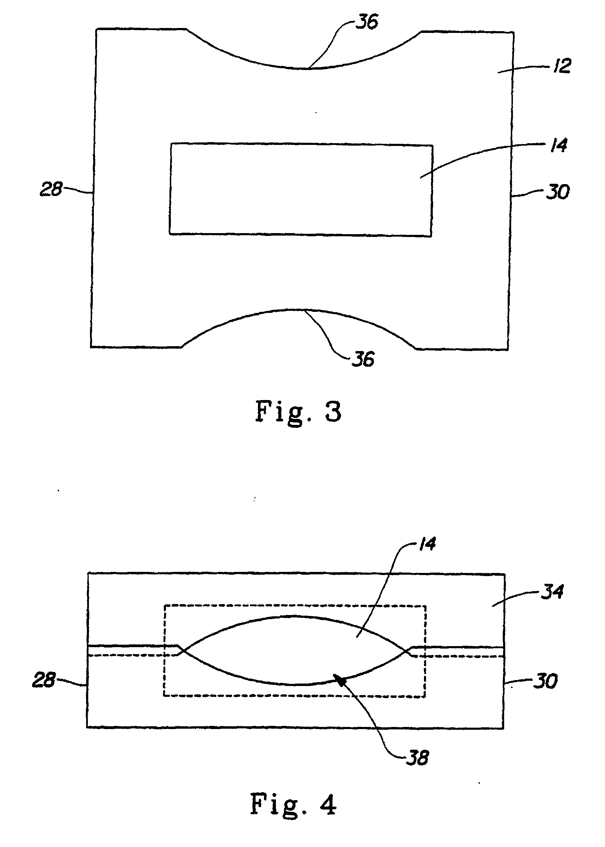

After the core 14 is secured in the center of the web 12, opposing sides of the web are folded toward the center, around the core, along fold lines 37, as shown in Figs. 1, 2, and 5, to form a top sheet 34 above the core. The opposing sides of the web 12 are secured together along the top surface of the diaper by additional bands of adhesive 24. In order to allow waste material to access the absorbent core 14, the web 12 is notched on opposing sides, prior to folding, as shown in Fig. 3, to form cutouts 36. When the web 12 is folded around the core 14, the cutouts 36 combine to define an opening 38 extending along the length of the top surface of the core, as shown in Figs. 1 and 4. The opening 38 provides access to the core 14, so that body fluids and waste materials from the wearer may pass through the web 12 to the acquisition layer and absorbent material below.

The cutouts 36 are preferably generally oblong, and, in the representative embodiment depicted in the figures, are shaped so as to form an elliptical opening in the web 12, when the web is folded about the core 14. The length of the cutouts 36 may vary depending upon the intended wearer and use of the garment, provided that the cutouts are of sufficient length to enable the opening 38, formed thereby, to extend at least substantially about the periphery of the genital area of the wearer, so that liquid and solid waste from the wearer comes in contact with the core 14. Since the web 12 is folded around the core 14 to produce a combined top and bottom sheet, rather than having separate top and bottom sheets in the diaper, there is no need for additional peripheral seams to join the sheets together. Eliminating the seams between the top and bottom sheets enables the diaper of the present invention to be stronger than conventional disposable diapers. Further, since the core 14 is surrounded by the liquid impermeable web 12 except for the opening 38 on the top surface of the core, body fluids are trapped within the web and cannot leak from the sides of the diaper, thereby increasing the leak resistance of the diaper.

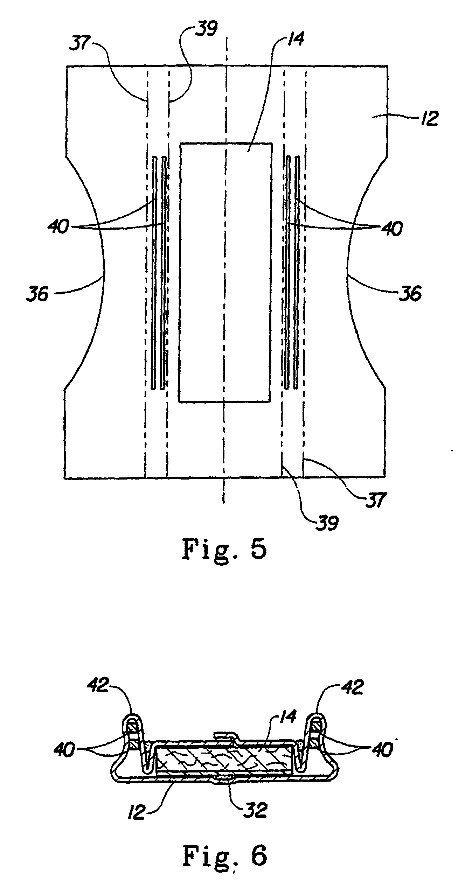

Before the web 12 is folded around the core 14, a resilient material is preferably attached to the web inward of the notches 36. In the diaper embodiment depicted in the figures, the resilient material is one or more rows of elastic bands 40, shown in Figs. 5 and 6, which are affixed to the web 12. The elastic bands 40 are preferably attached in the crotch portion 26 of the garment, adjacent and substantially parallel to each of the cutouts 36. In the representative embodiment, the elastic bands 40 are attached to the web 12 in a strained mode, so that, when permitted to relax, the elastic bands cause a portion of the web 12 to gather.

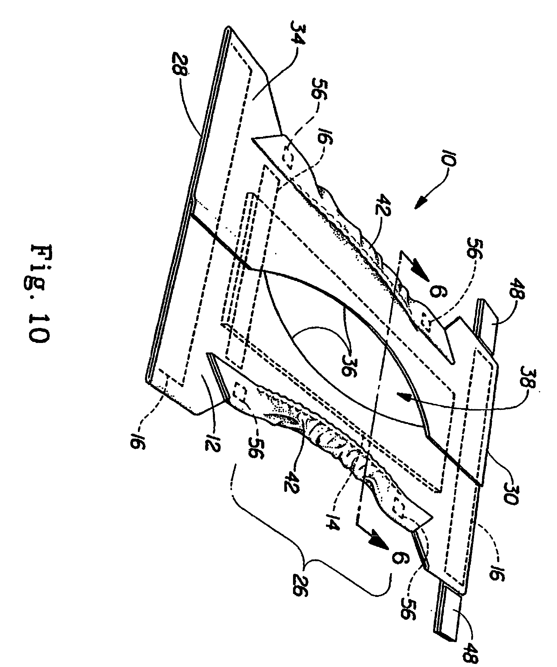

In order to form leg cuffs, the sides of the folded web 12 are folded again, through the crotch area 26, along secondary fold lines identified as dotted lines 39 in Fig. 5. In the preferred embodiment, the elastic bands and secondary fold lines are positioned on the web 12 such that the elastic bands are located within the folded portion of the web as shown in Fig. 6. To maintain the sides of the web 12 in a folded position, the folded web is tacked down at the ends of the crotch portion by an adhesive or other similar bonding technique as shown by attachment points 54 in Fig. 1. When the elastic bands 40 are permitted to relax, the elastic bands cause the side, folded portions of the web to gather and form the leg cuffs 42. Figure 10 depicts an alternative embodiment for the leg cuffs 42. In the alternative embodiment, the web 12 may be cut inwardly adjacent the ends of the folded portion, to form a separate, distinct cuff ear, and the cuff ear tacked down at the ends, as shown at attachment points 56, to form the leg cuffs.

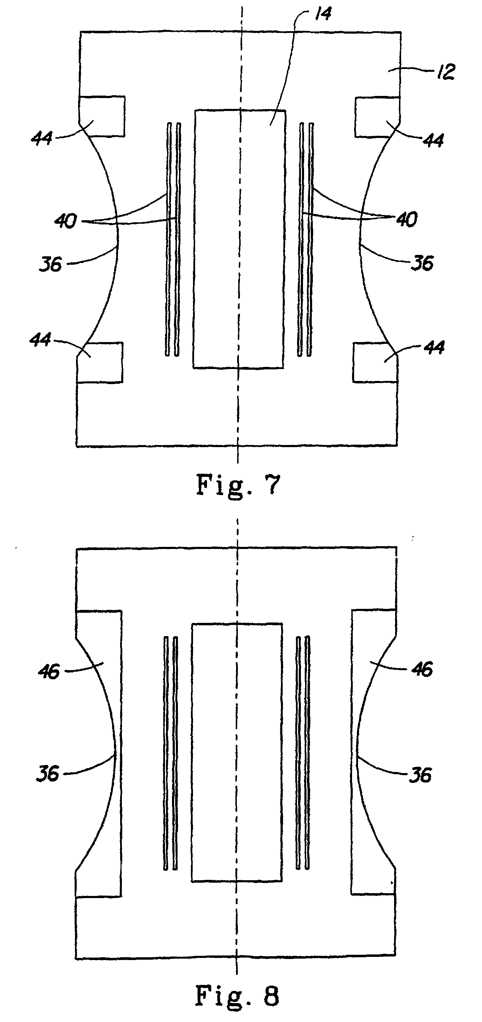

In one preferred embodiment of the present invention, a lifting arrangement is provided on the web 12 adjacent the opening 38. The lifting arrangement may, for example, comprise a heat shrinkable film or other similar material, which will cause the edges of the opening 38 to be raised slightly relative to the remaining surface of the web. Such a film may be applied as four patches 44 at the corners of the cutouts 36, such as shown in Fig. 7, so that when the web 12 is folded, the film is located at the ends of the opening 38. In the alternative, the film may be applied as two patches 46, one patch being located along the perimeter of each of the cutouts 36, as shown in Fig. 8, so that when the web 12 is folded around the core 14, the film extends about the entire opening 38. In either version, the film is preferably applied to the web 12 before notching and folding. When the film is heated, the film contracts, to raise either just the ends or the entire peripheral edge of the web 12, about the opening 38. The raised edges of the web 12 preferably form a containment area or "pocket" about the opening 38. This pocket helps to capture solid waste material in the opening area 38 and prevent the waste from traveling throughout the diaper and soiling other areas of the wearer. These lifting arrangements, provided as a plurality of selectively located but shrinkable structures, are provided only as preferred examples of the variety of structures which could be utilized to provide raised portions about the opening area 38. Other structural combinations will be apparent to those skilled in the art.

In order to secure the diaper about the wearer's torso during use, a closure device is preferably provided on the web 12. In the representative embodiment depicted in the drawings, the closure device comprises a pair of adhesive tabs 48 which are attached on opposing sides of the web 12. The adhesive tabs 48 can be releasably attachable to the corresponding front edge of the diaper, adjacent the waist portion 28, in a conventional manner, in order to properly fit and retain the diaper on the wearer. While the invention is described with respect to adhesive tabs as the closure device, it is to be understood that other releasable closure devices, such as Velcro® type tabs, could also be used without departing from the scope of the invention.

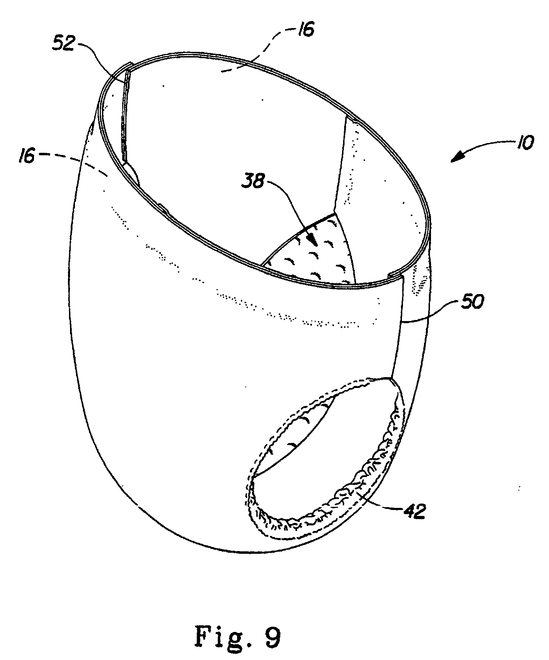

Figure 9 depicts an alternative embodiment for the garment of the invention. In the garment shown in Fig. 9, opposing sides are lap or butt welded along edges 50, 52 to form a pant. Welding the side edges together enables the garment to be used in a pull-up type capacity such as in a child's training pant. With the exception of the side welds, the garment of Fig. 9 is substantially the same as that shown in Figs. 1-8 with the same features and benefits.

In summary, the present invention provides a disposable garment in which a single carrier web is utilized as both a bottom and top sheet to eliminate the need for additional attachment seams about the periphery of the garment. By eliminating the seams, and surrounding the absorbent core with a liquid impervious web, the present invention provides a disposable garment that is simpler in construction and inherently stronger and more leak resistant than traditional disposable garments. In addition, because the garment of the present invention utilizes a single carrier web which is hydrophobic, body fluids are confined to the absorbent core rather than being wicked throughout the top sheet, as in conventional disposable garments, thereby eliminating leaks from the edges of the garment. Because the garment of the present invention includes a stretchable carrier web and elastomeric material for increasing the elastic force of the web, the present invention provides uniform stretch across the entire garment and better conformance to the shape of the wearer. Thus, the garment is more comfortable to wear and is less likely to gap and leak. Further, in the present invention, only the single carrier web is notched, rather than both a top and bottom sheet being notched as in previous disposable garment designs. Accordingly, less material is required to produce the garment and less material is wasted through the notching process, without negatively effecting the performance of the garment.

The foregoing description of a preferred embodiment of the invention has been presented for purposes of illustration and description. It is not intended to be exhaustive or to limit the invention to the precise form disclosed . The embodiments shown and described were chosen and described in order to best illustrate the principles of the invention and its practical application to thereby enable one of ordinary skill in the art to best utilize the invention in various embodiments and with various modifications as are suited to the particular use contemplated. It is intended that the scope of the invention be defined by the claims appended hereto.

## Claims

1. A disposable diaper (10) with a front waist margin (28), a rear waist margin (30), a crotch portion (26), and a longitudinal centerline, said diaper comprising:
an absorbent core (14);
**characterized by**
an impervious web (12) surrounding said core (14) and with overlapping seams at the front and rear waist margins (28, 30) of the diaper (10), said seams being juxtaposed with the centerline of the diaper, said web (12) being resiliently stretchable in a direction substantially perpendicular to said centerline and including an opening (38) along a top surface of said core (14), said opening (38) having a peripheral edge formed by cutouts (36) and being adapted to permit fecal materials from a wearer to pass to said core (14); and
at least one strip of elastomeric material (40) attached to the web (12) on each side of the centerline adjacent the opening (38) so as to extend at least partially across the crotch portion (26) of the diaper to provide leg cuffs along said crotch portion.

2. A diaper as claimed in Claim 1, further comprising a lifting arrangement (44) associated with the web adjacent said opening for raising at least a portion of the peripheral edge adjacent said opening, said lifting arrangement being disposed at least at opposite ends of the opening for forming a containment area, said lifting preferably comprising
a heat shrinkable film material disposed about at least part of the peripheral edge of the opening for raising at least a portion of said peripheral edge relative to said core (14);
a closure device (48) for attaching opposing sides of said diaper together about a wearer's body;
at least one strip of resilient material (14) for conforming said garment to said wearer's body, said resilient material being associated with said web so as to increase a return force of said web in a direction substantially perpendicular to said longitudinal centerline; and
preferably wherein said core (14) is attached to said web (12) by an adhesive material (32) centered between said web and said core.

3. A diaper as claimed in claims 1 and 2 wherein said lifting arrangement (44) is disposed about substantially the entire perimeter of said peripheral edge.

4. A diaper as claimed in claims 1 and 2 wherein said lifting arrangement (44) is disposed only at opposite ends of said opening.

5. The disposable diaper of claim 2 wherein said heat shrinkable film material (44) is disposed adjacent substantially the entire peripheral edge of said opening (38) for raising the edges of said opening (38).

6. A method of constructing a disposable diaper (10) with a front waist margin (28), a crotch portion (26), a rear waist margin (30) and a longitudinal centerline, said method **characterized by** the steps of:
providing a liquid impermeable carrier web (12) having a first surface and longitudinal sides;
applying at least one strip of an elastomeric material (40) to said first surface of the carrier web (12) so as to extend at least partially across said crotch portion (26) of the diaper (10) and substantially parallel with said centerline;
applying a heat shrinkable film (44) at selected locations along said longitudinal sides of the carrier web;
notching opposing longitudinal sides of said carrier web outward of said elastomeric material to form notches (36) therein;
providing an absorbent core (14);
attaching said absorbent core (14) to said web (12); and
folding said web (12) about said core (14) such that said notches (36) define an opening to said core, said web (12) being folded so as to overlap at seams located in said front and rear waist margins (28, 30) of said diaper (10) and secured together adjacent said seams, said seams being juxtaposed with the centerline of the diaper (10).

7. The method of claim 7 wherein said step of applying a heat shrinkable film effectively provides a lifting arrangement along opposing sides of said carrier web.

8. The method of claim 8 wherein after said folding step, said web is heated to cause said heat shrinkable film to provide a lifting arrangement to raise at least portions of said web adjacent said opening and relative to said core.

## Patentansprüche

1. Einwegwindel (10) mit einem vorderen Taillenrand (28), einem hinteren Taillenrand (30), einem Schrittbereich (26) und einer längs verlaufenden Mittellinie, wobei die Windel aufweist:
einen absorbierenden Kern (14);
**gekennzeichnet durch**
eine undurchlässige Bahn (12), welche den Kern (14) umgibt und mit überlappenden Nähten an dem vorderen und hinteren Taillenrand (28, 30) der Windel 10), wobei die Nähte an der Mittellinie der Windel liegen, wobei die Bahn (12) in einer Richtung im wesentlichen rechtwinklig zur Mittellinie elastisch dehnbar sind und eine Öffnung (38) entlang einer Oberseite des Kerns (14) enthalten, wobei die Öffnung (38) einen Umfangsrand haben, der **durch** Ausschnitte (36) gebildet wird und so angepaßt ist, daß Stuhlgangmaterialien von einem Träger zu dem Kern (14) gelangen können;
wenigstens einen Streifen eines elastomeren Materials (40), das an der Bahn (12) auf jeder Seite der Mittellinie angrenzend an die Öffnung (38) angebracht ist, so daß sich dieses wenigstens teilweise über den Schrittbereich (26) der Windel erstreckt, um Beinaufschläge entlang des Schrittbereichs zu schaffen.

2. Windel nach Anspruch 1, ferner mit einer Hebeanordnung (44), die mit der Bahn angrenzend an die Öffnung verbunden ist, um wenigstens einen Bereich des Umfangsrandes angrenzend an die Öffnung anzuheben, wobei die Hebeanordnung wenigstens an entgegengesetzten Enden der Öffnung zum Bilden einer Aufnahmefläche angeordnet ist, wobei die Hebeanordnung vorzugsweise aufweist
ein wärmeschrumpffähiges Filmmaterial, das um wenigstens einen Teil des Umfangsrandes der Öffnung zum Anheben wenigstens eines Bereichs des Umfangsrandes relativ zum Kern (14) angeordnet ist;
einer Schließeinrichtung (14) zum aneinander Anbringen von gegenüber liegenden Seiten der Windel um den Körper eines Trägers herum;
wenigstens einem Streifen eines elastischen Materials (16) zum Anschmiegen der Wäsche an den Körper des Trägers, wobei das elastische Material mit der Bahn so verbunden ist, daß dieses eine Rückkehrkraft der Bahn in einer Richtung im wesentlichen rechtwinklig zur längs verlaufenden Mittellinie steigert; und
wobei vorzugsweise der Kern (14) an der Bahn (12) durch ein Haftmaterial (32) angebracht ist, das zwischen der Bahn und dem Kern zentriert ist.

3. Windel nach den Ansprüchen 1 und 2, in welcher die Hebeanordung (44) um im wesentlichen den gesamten Umfang des Umfangsrandes angeordnet ist.

4. Windel nach den Ansprüchen 1 und 2, in welcher die Hebenanordnung (44) nur an gegenüber liegenden Enden der Öffnung angeordnet ist.

5. Einwegwindel nach Anspruch 2, in welcher das wärmeschrumpffähige Filmmaterial (44) angrenzend im wesentlichen an den gesamten Umfangsrand der Öffnung (38) zum Anheben der Ränder der Öffnung (38) angeordnet ist.

6. Verfahren zum Konstruieren einer Einwegwindel (10) mit einem vorderen Taillenrand (28), einem Schrittbereich (26), einem hinteren Taillenrand (30) und einer längs verlaufenden Mittellinie, wobei das Verfahren **gekennzeichnet ist durch** die Schritte:
Bereitstellen einer flüssigkeitsundurchlässigen Trägerbahn (52) mit einer ersten Oberfläche und längs verlaufenden Seiten;
Aufbringen wenigstens eines Streifens eines elastomeren Materials (40) auf die erste Oberfläche der Trägerbahn (12), um diese so wenigstens teilweise über den Schrittbereich (26) der Windel (10) und im wesentlichen parallel zur Mittellinie auszudehnen;
Aufbringen wenigstens eines schrumpffähigen Films (44) an ausgewählten Stellen entlang der längs verlaufenden Seiten der Trägerbahn:
Ausschneiden gegenüberliegender Längsseiten der Trägerbahn außerhalb des elastomeren Materials, um Ausnehmungen (36) darin auszubilden;
Bereitstellen eines absorbierenden Kerns (14);
Anbringen des absorbierenden Kerns (14) an der Bahn (12); und
Falten der Bahn (12) um den Kern (14), derart, daß die Ausnehmungen (36) eine Öffnung zum Kern begrenzen, wobei die Bahn (42) so gefaltet wird, daß diese an Nähten überlappen, die in dem vorderen und hinteren Taillenrand (28, 30) der Windel (10) liegen und angrenzend an die Nähte aneinander festgelegt werden, wobei die Nähte an der Mittellinie der Windel (10) liegen.

7. Verfahren nach Anspruch 7, in welchem der Schritt des Aufbringens eines wärmeschrumpffähigen Films eine Hebeanordnung entlang gegenüber liegender Seiten der Trägerbahn wirksam liefert.

8. Verfahren nach Anspruch 8, in welchem nach dem Faltungsschritt die Bahn erwärmt wird, um den wärmeschrumpffähigen Film zu veranlassen, eine Hebeanordnung zu schaffen, um wenigstens Bereiche der Bahn angrenzend an die Öffnung und relativ zum Kern anzuheben.

## Revendications

1. Couche jetable (10) ayant une bordure de ceinture avant (28), une bordure de ceinture arrière (30), une partie d'entrejambe (26), et une ligne médiane longitudinale, ladite couche comprenant :
une âme absorbante (14) ;
**caractérisée par** :
une nappe imperméable (12) entourant ladite âme (14) et ayant des coutures en chevauchement au niveau des bordures de ceinture avant et arrière (28, 30), lesdites coutures étant juxtaposées à la ligne médiane de la couche, ladite nappe (12) pouvant être étirée de manière élastique dans une direction sensiblement perpendiculaire à ladite ligne médiane, et comprenant une ouverture (38) sur une surface supérieure de ladite âme (14), ladite ouverture (38) ayant un bord périphérique formé par des découpes (36) et étant adaptée pour permettre aux matières fécales provenant d'un utilisateur de passer vers ladite âme absorbante (14) ; et
au moins une bande de matériau élastomère (40) attachée à la nappe (12) de chaque côté de la ligne médiane, en position adjacente de l'ouverture (38), de façon à s'étendre au moins partiellement à travers la partie d'entrejambe (26) de la couche pour réaliser des revers de jambe le long de ladite partie d'entrejambe.

2. Couche selon la revendication 1, comprenant, en outre, un agencement de soulèvement (44) associé à la nappe en position adjacente de ladite ouverture, pour soulever au moins une partie du bord périphérique adjacent à ladite ouverture, ledit agencement de soulèvement étant disposé au moins à des extrémités opposées de l'ouverture, pour former une région de retenue, ledit agencement de soulèvement comprenant, de préférence :
un film rétractable à la chaleur disposé autour d'au moins une partie du bord périphérique de l'ouverture, pour soulever au moins une partie dudit bord périphérique par rapport à ladite âme (14) ;
un dispositif de fermeture (48) pour attacher ensemble les côtés opposés de ladite couche autour du corps d'un utilisateur ;
au moins une bande de matériau élastique (16) pour que ledit vêtement épouse le corps dudit utilisateur, ledit matériau élastique étant associé à ladite nappe de façon à augmenter une force de rappel exercée par ladite nappe dans une direction sensiblement perpendiculaire à ladite ligne médiane longitudinale,
ladite âme (14) étant, de préférence, attachée à ladite nappe (12) par un matériau adhésif (32) centré entre ladite nappe et ladite âme.

3. Couche selon les revendications 1 et 2, dans laquelle ledit agencement de soulèvement (44) est disposé de pratiquement tout le périmètre dudit bord périphérique.

4. Couche selon les revendications 1 et 2, dans lequel ledit agencement de soulèvement (44) est disposé uniquement à des extrémités opposées de ladite ouverture.

5. Couche jetable selon la revendication 2, dans laquelle ledit film rétractable à la chaleur (44) est disposé en position adjacente de pratiquement tout le bord périphérique de ladite ouverture (38), pour soulever les bords de ladite ouverture (38).

6. Procédé de construction d'une couche jetable (10) ayant une bordure de ceinture avant (28), une partie d'entrejambe (26), une bordure de ceinture arrière (30), et une ligne médiane longitudinale, ledit procédé étant **caractérisé par** les étapes qui consistent à :
prévoir une nappe porteuse (12) imperméable aux liquides, ayant une première surface et des bords longitudinaux ;
appliquer au moins une bande d'un matériau élastomère (40) sur ladite première surface de la nappe porteuse (12), de façon qu'elle s'étende au moins partiellement à travers ladite partie d'entrejambe (26) de la couche (10) et dans une direction sensiblement parallèle à ladite ligne médiane ;
appliquer un film rétractable à la chaleur (44) à des endroits choisis le long desdits bords longitudinaux de la nappe porteuse ;
encocher les côtés longitudinaux opposés de ladite nappe porteuse à l'extérieur dudit matériau élastomère pour y former des encoches (36) ;
prévoir une âme absorbante (14) ;
attacher ladite âme absorbante (14) à ladite nappe (12) ; et
replier ladite nappe (12) autour de ladite âme (14) de telle sorte que lesdites encoches (36) définissent une ouverture vers ladite âme, ladite nappe (12) étant repliée de façon à se chevaucher au niveau de coutures situées dans lesdites bordures de ceinture avant et arrière (28, 30) de ladite couche (10) et étant fixée en position adjacente desdites coutures, lesdites coutures étant juxtaposées à la ligne médiane de la couche (10).

7. Procédé selon la revendication 7, dans lequel ladite étape qui consiste à appliquer un film rétractable à la chaleur réalise un agencement de soulèvement efficace le long des côtés opposés de ladite nappe porteuse.

8. Procédé selon la revendication 8, dans lequel, après l'étape de pliage, ladite nappe est chauffée pour que ledit film rétractable à la chaleur puisse réaliser un agencement de soulèvement, pour soulever au moins des parties de ladite nappe, adjacentes de ladite ouverture et par rapport à ladite âme.
